# EUROPEAN PATENT APPLICATION

(11) **EP 1 129 663 A1**
(43) Date of publication of application: **05.09.2001**
(21) Application number: 01301893.2
(22) Date of filing: 01.03.2001
(51) Int. Cl.: A61B 6/00

(54) **Method and system for positioning a radiology apparatus**

(30) Priority: 01.03.2000 FR 0002648
(71) Applicant: GE MEDICAL SYSTEMS SA, 78533 Buc Cedex (FR)
(72) Inventor: Lienard, Jean, 92140 Clamart (FR); Boutenko, Vladislav, 92100 Boulogne-Billancourt (FR); Belanger, Barry Frederick, Salt Lake City, Utah 84124 (US)
(74) Representative: Pedder, James Cuthbert

(57) **Abstract**

A method and system for automatic positioning of a radiology apparatus the type comprising an X-ray emission means capable of emitting an X-ray beam in a so-called viewing direction, and an X-ray reception means, the positioning being carried out in relation to an object to be X-rayed, in which a first direction is determined, a first cone angle of displacement relative to the first direction that is less than the first angle is determined, the viewing direction being calculated so that the image quality will be superior to what would be obtained with a viewing direction parallel to the first direction.

## Description

The present invention is directed to a method and an apparatus for radiology and, in particular, the positioning of such an apparatus.

A radiology apparatus for mammographic, RAD or RF conventional radiology, neurological or even vascular (peripheral or cardiac) use, for example, generally comprises: (1) an X-ray tube and a collimator for forming and delimiting an X-ray beam; (2) an image receiver, generally a radiological image intensifier and a video camera, or even a solid state detector; (3) a positioner supporting the X-ray tube and collimator assembly, on one side, and image receiver, on the other, movable in space around one or more axes; and (4) a means of positioning an object, e.g., a patient, such as a table provided with a platform intended to support the patient in an elongated position.

The radiology apparatus further comprises means for control of the X-ray tube making it possible to adjust the parameters such as X-radiation dose, exposure time, high voltage, etc.; a means for control of the different motors making it possible to displace the radiology apparatus on its different axes; means for positioning the patient and means for image processing making possible an on-screen display and data storage for two- or three-dimensional images with functions such as a zoom, a translation along one or more perpendicular axes, a rotation on different axes, a subtraction of images or even a contour retrieval. These functions are secured by electronic charts capable of different adjustments.

A method for acquisition of images of a body by rotational positioning is known according to patent FR-A-2,705,224. As disclosed in FR-A-2,705,224, the conicity of the X-ray beam, the measurements taken to quantify a lesion observed on an image, on an angiographic examination, for example, are correct only if the local direction of the vessel considered is parallel to the plane of the detector and the quality of the display and quantification of the lesions strongly depends on choice of the acquisition incidence angles. The possibility of positioning the plane of the detector of the apparatus parallel to the main axis of a vessel enables the vessel to be visualizes under the best conditions. FR-A-2,705,224 uses two reference images, acquired under two difference angles of incidence, in order to determine automatically the three-dimensional orientation of the vessel of interest. In a three-axis device, the angular positions of the first two axes are determined in order to place the third axis parallel to the vessel. Rotation on the third axis is then freely used in order to make acquisitions. Thus, the spatial positions of the focus of the X-ray tube and detector are located, two images of the body containing the vessel are acquired for two positions of the device, a first point representative of a first locus characteristic of the direction of the vessel is targeted and located on a first image and in space, the spatial coordinates of a line of projection passing through that first point and the X-ray tube focus are calculated, an epipolar line, image of that line of projection, is represented in the second image, a point homologous with the first point is targeted and marked in that second image and in space on the epipolar line, the position in space of the first characteristic locus is deduced from the locations of the points in the two images, these operations are repeated for a second characteristic locus, the direction of the vessel is deduced therefrom, and the rotating image or images are then acquired by having one or more positions rotating on an axis roughly collinear with that of the vessel occupied in the device. The center axis of the X-ray beam is then perpendicular to the direction of the vessel.

This method has proven satisfactory. However, the need has appeared to obtain images of good quality and homogeneous quality, whatever the angulation adopted.

The present invention is directed to a method and system for positioning of a radiology apparatus to obtain optimal quality images.

The present invention concerns a method and system for positioning of a radiology apparatus introducing an advantageous compromise between an optimal angulation making precise measurements possible and an angulation making an optimal image quality possible.

The method and system for automatic positioning, according to one aspect of the invention, is intended for a radiology apparatus of the type comprising means for X-ray emission and a means for X-ray reception. The means for emission is capable of emitting an X-ray beam in an adjustable so-called viewing direction, the positioning being carried out in relation to an object to be X-rayed. A first direction is determined, a first cone angle relative to the first direction is determined, and a viewing direction presenting an angle of displacement relative to the first direction that is less than the first angle is determined, the viewing direction being calculated so that the image quality will be superior to what would be obtained with a viewing direction parallel to the first direction. The optimal quality of an image is defined by the adequacy of the dynamics of the image signals on input of the X-ray detector and of the intrinsic dynamics of the X-ray detector.

The invention will now be described in greater detail, by way of example, with reference to the drawings, in which:-
Figure 1 is a view in perspective of a radiology apparatus with three axes;
Figure 2 is a schematic view showing a possible angulation;
Figure 3 is a schematic view showing the maximum of the possible angulations;
Figure 4 is a diagram showing a stage of the method; and
Figure 5 is a view in perspective illustrating the method.

The invention is directed to a system of automatic positioning of a radiology apparatus of the type including a means for X-ray emission and a means for X-ray reception, the means for emission being capable of emitting an X-ray beam in an adjustable so-called viewing direction, the positioning being carried out in relation to an object to be X-rayed. The system includes a means for determining a first direction, a means for determining a first cone angle relative to the first direction, and a means for determining a viewing direction presenting an angle of displacement relative to the first direction that is less than the first angle, the viewing direction being calculated so that the image quality will be superior to that which would be obtained with a viewing direction parallel to the first direction.

The invention is directed to a system of automatic positioning of a radiology apparatus of the type comprising an X-ray emitter capable of emitting an X-ray beam in a so-called viewing direction, an X-ray receiver, a positioner capable of moving the emitter and receiver in relation to an object to be X-rayed, and a control unit. The system comprises a means for determining a first direction, a means for determining a first cone angle α relative to the direction, and a means for determining a viewing direction presenting an angle of displacement β relative to the first direction less than the first angle α, the viewing direction being calculated so that the image quality is superior to that which would be obtained with a viewing direction parallel to the first direction.

The invention also concerns a radiology apparatus including a positioning system, such as above.

Thus, an initial positioning is carried out, on the basis either of a standard positioning, for example, of frontal or anterior oblique type, or of a positioning to which the operator is accustomed, or even of an optimal positioning determined by a method of optimization of positioning, such as proposed by Patent FR-A-2,705,224. Simultaneously, and within the angular limits determined, in relation to the first direction, the system determines an angulation which offers an image quality improved over that obtained parallel to the first direction. The angulation making possible an improvement of image quality can be determined by means of information on the radiological thickness of the patient as a function of angulation thanks to images previously acquired, by means of analysis of the dynamics of images previously acquired, by means of a three-dimensional model of the patient constructed from previously acquired X-ray images, or even by means of a combination of the possibilities envisaged above.

Angulation is understood here to be a set of n values of angles making it possible to define the position in space of the X-ray beam; n is generally equal to 3, but can also be equal to the number of axes of rotation of the apparatus, which can be different from 3, for example, 2 or 4.

The shortening of an object in a spatial direction perpendicular to the first direction, which can be observed on the image by reason of deviation of the X-ray beam from the first direction, can be controlled by the operator. It is possible to know in advance the maximum error which might be due to that deviation, when a lesion is measured or, more generally, any length of an object present on the image.

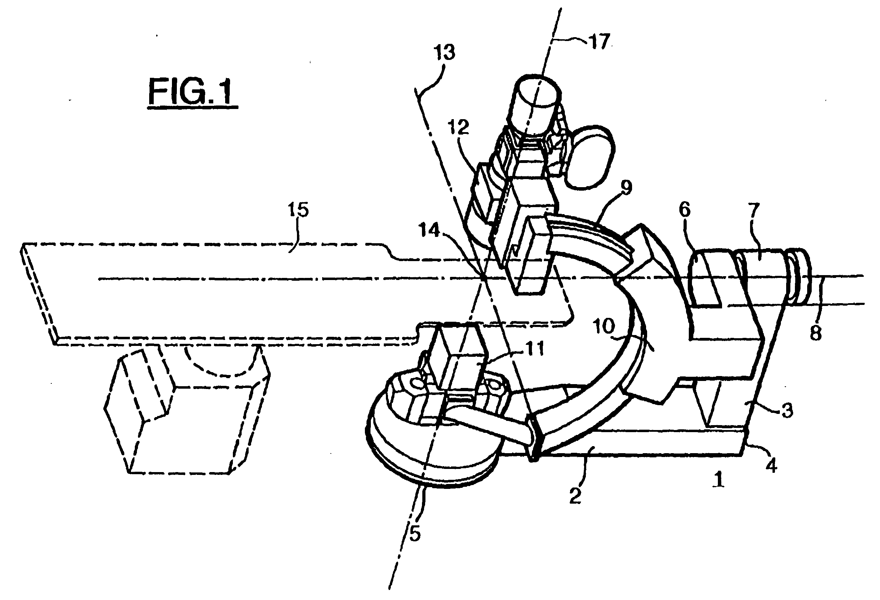

As can be seen in Figure 1, a radiology apparatus contains an L-shaped pedestal 1, with a roughly horizontal base 2 and a roughly vertical support 3 fastened to an end 4 of the base 2. At the opposite end 5, the base 2 contains an axis of rotation parallel to the support 3 and on which the pedestal is capable of turning. A support arm 6 is fastened by a first end to the top 7 of the support 3, rotating on an axis 8. The support arm 6 can take the shape of a bayonet. A C-shaped circular arm is maintained by another end 10 of the support arm 6. The C-shaped arm 9 is capable of sliding rotating on an axis 13 in relation to the end 10 of the support arm 6. A control unit, not represented, equipped with memory means and software means, is provided.

The C-shaped arm 9 supports an X-ray tube 11 and an X-ray detector 12 in diametrically opposite positions facing each other. The detector 12 contains a flat detection surface. The direction of the X-ray beam is determined by an axis 17 joining a focal point of the tube 11 to the center of the flat surface of the detector 12. The vertical axis of rotation of the pedestal 1, the axis 8 of the support arm 6 and the axis 13 of the C-shaped arm 9 are secant at a point 14. In mid-position, these three axes are mutually perpendicular. Axis 17 passes through point 14. A table 15, provided to accommodate a patient, possesses a longitudinal orientation aligned with axis 8 in rest position.

A set of images of a blood vessel is acquired for different positions of the radiology apparatus. The three-dimensional image is then reconstructed from a set of two-dimensional images. Two-dimensional views of the three-dimensional image are produced interactively, by virtually turning the three-dimensional image of the vessel in all desired directions until the view enabling the operator to best visualize the object of interest, a vessel portion, for example, is obtained. A vessel portion is an object extended along an axis.

Patent FR-A-2,705,224 discloses obtaining images at an optimal angulation by positioning relative to a longitudinal axis 18 a portion of the blood vessel it is desired to study. This portion is acquired at two different angulations as a result of which the longitudinal axis is determined. The radiology apparatus, which will be of three or four-axis type, is then positioned, so that rotation in relation to only one axis of the machine is used. In other words, the radiology apparatus is so controlled that the axis 17 of the X-ray beam is perpendicular to the longitudinal axis 18 of the vessel portion.

However, this angulation makes possible very precise measurement of elements of interest present on the image thanks to the orthogonality between axis 17 of the X-ray beam and the longitudinal axis 18 of the vessel portion, but not necessarily the best image that can be obtained in terms of image quality. For example, if too great or too small a thickness of radio-absorbent material is present along the axis 17 of the X-ray beam and attenuates the beam excessively or insufficiently.

With knowledge of the equivalent thickness of the patient around the vessel portion - in other words, with knowledge of the attenuation sustained by the X-ray beam -, it is desirable to find an angulation which, while making possible a precise measurement of the elements represented on the image, supplies a high image quality.

With a particular angulation, a first cone angle is going to be defined, which constitutes the maximum deviation allowed from the angulation. That first angle or maximum angle α defines a cone whose apex is point 14 and whose axis 19 is perpendicular to the longitudinal axis 18 of a vessel portion or of an object. However, the axis 19 of the cone can also be defined in relation to one of the axes of the radiology apparatus. In fact, users may wish to utilize a standard angulation, or an angulation they are accustomed to using and might memorize, for example, in a control means of the radiology apparatus.

The maximum angle α depends on the position of the X-ray tube, the angulation, the field and the distance between the X-ray tube and the detector. These variables are known with a degree of imprecision. By combining the respective inaccuracies of those variables by addition, for example, a value of the maximum angle can be deduced. For example, a maximum angle α in the order of 10°, and even of 20° and possibly 30°, can be envisaged. The maximum angle α can also be determined by a method of calculation of the maximum error in relation to the longitudinal axis of the vessel portion, which calculation can be carried out with software used by a computer or a work station associated with the radiology apparatus.

Once the maximum angle α is determined, the maximum shortening of a structure of length L measured on an image taken along an axis included in the cone can be deduced therefrom. If the maximum angle is 10°, the maximum shortening is equal to (1 - cos 10°) x L.

Once the maximum angle α is determined, the angulation offering the best possible image quality is sought. That angulation corresponds to an angle of displacement β less than the maximum angle α and, therefore, a shortening less than or equal to 1 - cos β. The axis of the beam is then axis 17. This search for better image quality can be carried out with the aid of a three-dimensional thickness model of the patient undergoing radiological examination. The radiological thickness is determined from measurement of the local intensity of the X-ray beam reaching the detector. In fact, for a given zone of the image where the intensity is homogeneous, the logarithm of the intensity is proportional to the thickness to a near constant. This constant is easily determined from two measurements of intensity made for two different zones of thicknesses. The different relative local thicknesses are obtained in an image taken at a given angulation. Users can then be offered a particular angulation for which the vessel portion or object they wish to study in detail lies in a zone of optimal equivalent thickness - in other words, a zone of intensities received on the image adapted to the dynamics of the detector. As a variant, the radiology apparatus can be directly positioned in the angle α making possible an improvement of image quality.

However, it is noted that when the axis of sight 17 lies in the plane defined by the longitudinal axis 18 and by axis 19 of the cone, the shortening along the longitudinal axis 18 of the structures observed on an image taken along the axis of sight 17 is equal to 1 - cos β. If the axis of sight 17 lies in a plane perpendicular to the longitudinal axis 18 and passing through axis 19 of the cone, the shortening is nil, for the axis of sight 17 is perpendicular to the longitudinal axis 18. In a variant, one can then allow a displacement of maximum angle α in the plane defined by axes 18 and 19 and of maximum angle δ in the plane perpendicular to axis 18, with δ > α. The directrix of the cone is then a rectangle. By extrapolating if δ = 180° is chosen, which means not limiting the displacement, the maximum shortening is always equal to 1 - cos α, but the type of view and, therefore, the structures observed on the image will be different from those desired by the user. It will then be advisable to maintain a reasonable value for δ.

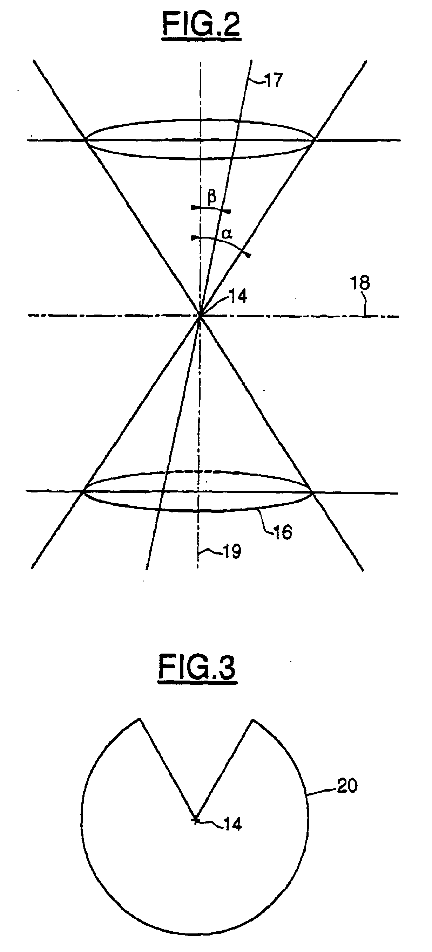

In Figure 2, the directrix of the cone is circle 16. It can also be arranged for the cone not to be generated by a directrix that is a circle, which corresponds to a constant maximum angle, but by an ellipse or other type of closed curve depending on the morphology of the part of the patient's body that is going to be visualized or even on limitation in angular movement of the parts of the radiology apparatus. In the latter case, the directrix can take the shape of a circle truncated on an angular sector, like the directrix 20 of Figure 3.

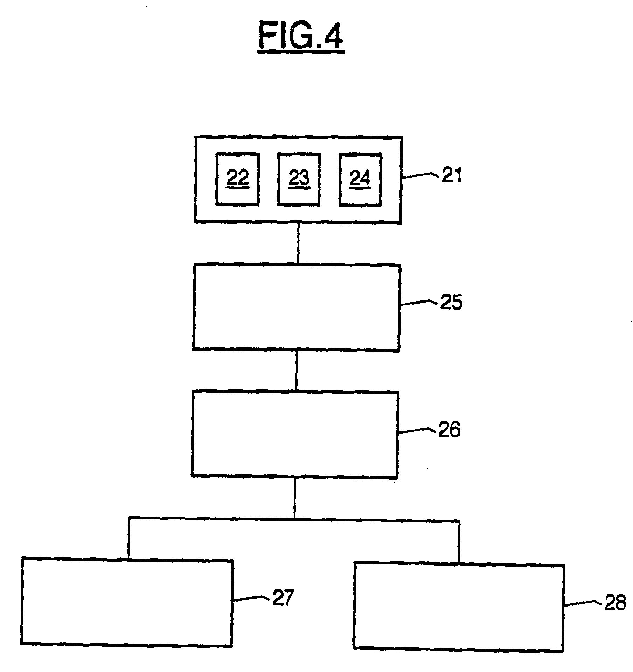

The operating stages are illustrated in Figure 4. In stage 21, the preferred angulation is chosen, which determines the axis 19 of the cone. This choice can be made according to at least three modalities:
(1) under stage 22, optimal angulation calculated according to Patent FR-A-2,705,224,
(2) under stage 23, standard angulation, frontal, for example,
(3) under stage 24, angulation memorized in a memory of the calculation unit.

In stage 25, the maximum tolerance of shortening of the structures observed is determined by reading of a value in a memory or by indication of a value by an operator. Angle α is deduced therefrom and, if necessary, δ.

In stage 26, the optimum radiological thickness is determined by keeping the axis 17 in the cone, resorting to a three-dimensional model, or to a calculation of average radiological thickness, or to an analysis of the image dynamics.

In stage 27, the angulation corresponding to the optimum previously determined is displayed. In stage 28, the radiology apparatus is positioned according to that angulation.

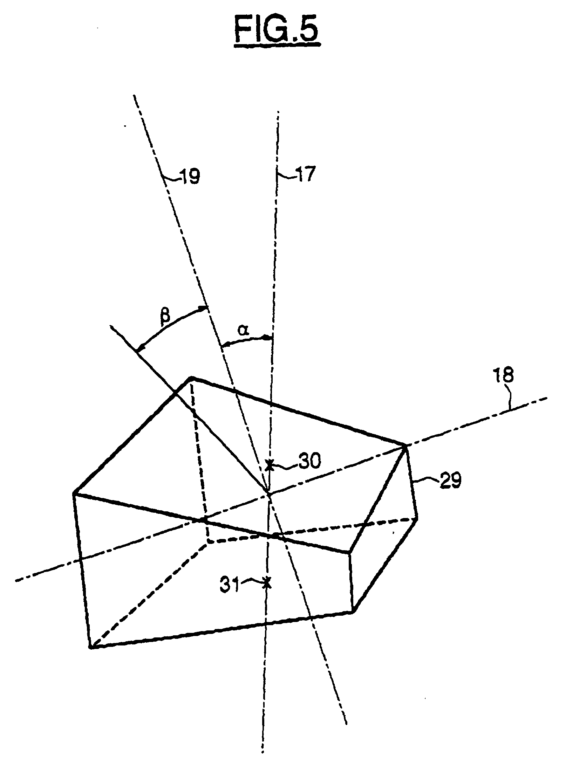

In Figure 5, a simplified model 29 of the radiological thickness of a part of the patient is represented. The axis 18 of the object to be studied has been determined by calculation, reading in a memory or approximation by the user. The axis 19 of the cone is perpendicular to axis 18. Angle α has been determined. The angle of sight 17 along which the X-rays cross the optimal radiological thickness for image quality was then sought. Here, angle β between axes 17 and 19 is close to angle α.

The intersection of the model 29 and the axis of sight 17 determines a point of entry 30 and a point of exit 31. The segment formed between those points 30 and 31 corresponds to the radiological thickness crossed by the X-ray beam. It can be seen that the radiological thickness is less if the X-ray beam is emitted along the axis of sight 17 than if it was along the axis 19 of the cone.

Thus, the angle of displacement can be generated from a three-dimensional model of the patient, a three-dimensional model constructed from previously acquired images or by analysis of the dynamics of such images, or even by using information relating to the radiological thickness of the patient as a function of angulation, which information can be supplied by a means of control of the radiology apparatus from previously acquired fluoroscopic or graphic images, for example.

The directrix of the cone in which the viewing direction is found is advantageously a circle or a rectangle.

In one embodiment, the first direction is perpendicular to a longitudinal direction of the object to be X-rayed.

In an embodiment of the invention, the direction of the object emanates from a value stored in a memory.

In an embodiment of the invention, the direction of the object emanates from a value calculated on a radiographic examination carried out previously.

In an embodiment of the invention, the direction of the object is obtained by calculation of the direction of a segment of the object acquired under at least two different viewing directions of the radiology apparatus.

In an embodiment of the invention, the first angle is obtained by calculation of a tolerance on the direction of the object. The first angle can be obtained by calculation of a maximum tolerance on the direction of the object.

The value of the first cone angle depends preferably on the position of the generatrix of the cone relative to the first direction in a three-dimensional frame of reference.

In an embodiment of the invention, the average radiological thickness of the object is calculated by means of images taken previously, for example, fluoroscopic or graphic images.

In an embodiment of the invention, the average radiological thickness of the object is calculated by means of a three-dimensional model of the object.

The average radiological thickness of the object is advantageously calculated by analysis of the dynamics of previously taken images.

In an embodiment of the invention, a preferred radiological imaging angle is calculated. The radiology apparatus can be automatically positioned at the preferred angle. A radiological image can be automatically taken at the preferred angle.

Thus, the invention makes it possible to obtain an excellent compromise between angulation and image quality automatically and with an angulation very close to that expected by the user. The image quality is significantly improved over what can be achieved for an angulation obtained according to the known methods. Shortening of the structures observed on the image is, in any case, very slight and can advantageously come within the margin of imprecision on length of structure observed on images for an angulation obtained in known fashion.

In any case, it will be planned to indicate to users the maximum relative error of length they will be likely to encounter when taking a measurement on the image, for example, a measurement of a lesion or a measurement concerning placement of a tool, such as a blood vessel prosthesis ("stent" in English).

The productivity of a user such as a cardiologist or a radiologist is increased through the possible automation of choice of angulation. Image quality will be enhanced, which will make possible an easier and more rapid interpretation by the user of information present on the image.

## Claims

1. A method for positioning of a radiology apparatus having means for X-ray emission and means for X-ray reception, the means for emission being capable of emitting an X-ray beam in an adjustable so-called viewing direction, the positioning being carried out in relation to an object to be X-rayed comprising the steps of:
a first direction is determined,
a first cone angle α relative to the first direction is determined,
and a viewing direction presenting an angle of displacement β relative to the first direction that is less than the first angle α is determined, the viewing direction being calculated so that the image quality will be superior to what would be obtained with a viewing direction parallel to the first direction.

2. The method according to claim 1, in which the directrix of the cone in which the viewing direction is found is a circle or a rectangle.

3. The method according to claim 1 or 2, in which the first direction is perpendicular to a longitudinal direction of the object to be X-rayed.

4. The method according to claim 2 or 3, in which the direction of the object is obtained by calculation of the direction of a segment of the object acquired under at least two different viewing directions of the radiology apparatus.

5. The method according to claim 3 or 4, in which the first angle is obtained by calculation of an error on the direction of the object.

6. The method according to any preceding claim, in which a preferred radiological imaging angle is calculated for automatically positioning the radiology apparatus at the angle or for automatically taking a radiological image at the preferred angle.

7. A system for positioning of a radiology apparatus comprising means for X-ray emission and means for X-ray, the means for emission being capable of emitting an X-ray beam in an adjustable so-called viewing direction, the positioning being carried out in relation to an object to be X-rayed, means for determining a first direction, means for determining a first cone angle α relative to the direction, and a means for determining a viewing direction presenting an angle of displacement β relative to the first direction that is less than the first angle α, the viewing direction being calculated so that the image quality will be superior to that which would be obtained with a viewing direction parallel to the first direction.

8. A system for positioning of a radiology apparatus comprising an X-ray emitter capable of emitting an X-ray beam in a so-called viewing direction, an X-ray receiver, a positioner capable of moving the emitter and receiver in relation to an object to be X-rayed, and a control unit, a means for determining a first direction, a means for determining a first cone angle α relative to the direction, and means for determining a viewing direction presenting an angle of displacement β relative to the first direction less than the first angle α, the viewing direction being calculated so that the image quality is superior to that which would be obtained with a viewing direction parallel to the first direction.

9. A radiology apparatus including a positioning system according to claim 7 or 8.
